# EUROPEAN PATENT APPLICATION

(11) **EP 1 382 692 A2**
(43) Date of publication of application: **21.01.2004**
(21) Application number: 03016011.3
(22) Date of filing: 15.07.2003
(51) Int. Cl.: C12Q 1/37

(54) **Enzymatic assay of HIV protease inhibitors**

(30) Priority: 18.07.2002 US 200096
(71) Applicant: Roche Diagnostics GmbH, 68305 Mannheim (DE); F. HOFFMANN-LA ROCHE AG, 4070 Basel (CH)
(72) Inventor: Arabshahi, Lili, Carmel, IN 46032 (US); Li, Haijuan, Fishers, IN 46038 (US)

(57) **Abstract**

A method of quantifying an HIV protease inhibitor in a biological sample includes combining HIV protease, a spectroscopic substrate for HIV protease, and a biological sample suspected of containing an HIV protease inhibitor to form an assay mixture. A spectroscopic property of the assay mixture may be measured and related to the concentration of the HIV protease inhibitor in the biological sample.

## Description

The clinical care of patients having acquired immunodeficiency disease syndrome (AIDS) has been substantially improved by the introduction of compounds which function as potent and specific HIV protease inhibitors. It is believed that the human immunodeficiency virus (HIV) is the causative agent responsible for AIDS, and that the enzyme HIV protease is responsible for catalyzing specific cleavages in the *gag* and *gag-pol* polypeptides of the HIV. A virus that synthesizes a mutationally inactivated HIV protease does not generally form infectious virions. HIV protease is thus an important target for which drugs against AIDS can be designed. HIV protease inhibitor compounds, as well as anti-HIV protease antibodies, can cause a reduction or cessation of the activity of HIV protease.

Currently, there are six HIV protease inhibitor compounds approved by the Food and Drug Administration (FDA) for treatment of AIDS patients - amprenavir, indinavir, lopinavir, nelfinavir, ritonavir, and saquinavir. Combination therapies involving HIV protease inhibitor compounds and HIV reverse transcriptase are the cornerstones of currently recommended therapies for HIV infection. Not all AIDS patients show the same optimal response to a combination therapeutic regimen. There can be a large variability in drug response between individual patients, and relationships between systemic exposure to protease inhibitor compounds and antiviral effect have been supported by accumulating clinical information. When a combination therapeutic regimen is administered to a patient, potential pharmacokinetic drug-drug interactions can improve or weaken the treatment. Patient compliance, which directly relates to maintenance of adequate drug levels, may also affect the outcome of the treatment.

It is thus desirable to measure concentrations of HIV protease inhibitor compounds in patients to ensure that drug exposure is sufficient to maintain antiviral activity in AIDS patients. In addition, the quantitative measurement of HIV protease inhibitor compounds in biological samples from test subjects is crucial in the drug development process. Other measurements of interest in the treatment of HIV include the measurement of anti-HIV protease antibodies in patient samples. The presence of anti-HIV protease antibodies indicates infection of the patient by HIV, and the detection of the antibodies can therefore be used to diagnose possible infection.

Typically, HIV protease inhibitor compounds in patient samples have been quantified by discontinuous assay methods. A discontinuous method provides for a single determination of the concentration of one or more compounds at a specific time point. A determination of changes in the concentration over time requires the separate analysis of multiple samples from distinct time intervals. For example, plasma samples can be analyzed for numerous HIV protease inhibitor compounds simultaneously using chromatographic methods. See, for example, Poirier, J.M. et al., *Ther. Drug Monit.* 22:465-473 (2000); Marzonlini, C., et al., J. *Chromatogr.* 740:43-58 (2000); and Remmel, R.P. et al., *Clin. Chem.* 46(1):73-81 (2000). The plasma samples are typically subjected to solid-phase extraction procedures prior to examination. Thus, the plasma is not analyzed directly but must be modified, adding complexity and expense to the analysis.

Another class of discontinuous assays is radioimmunoassays. For example, a derivative of an HIV protease inhibitor compound labeled with iodine-125 is reported to compete for binding with antibodies for the compound with any inhibitor compound in patient plasma. The precipitated antibody complexes are then analyzed for their level of radioactivity to determine the concentration of the HIV protease inhibitor compound. See, for example, Wiltshire, H.R. et al. *Analyt. Biochem.* 281:105-114 (2000).

In another example of a discontinuous assay, the presence of anti-HIV protease antibodies in plasma reportedly can be determined qualitatively by measuring the activity of HIV protease which is added to the plasma sample. For example, HIV protease can be added to the sample together with a substrate for HIV protease. Cleavage of the substrate can be measured by an immunoassay utilizing an antibody which specifically binds the cleavage products. An absence of cleavage products indicates the presence of anti-HIV protease antibodies. See, for example, U.S. Patent No. 5,171,662.

All of the above discontinuous methods for measuring inhibitor compounds and/or antibodies for HIV protease have met with mixed success. There is thus a need for an improved method to quantify HIV protease inhibitor compounds and/or anti-HIV protease antibodies in a biological sample. It is desirable that such a method can be easily carried out on currently available analytical instrumentation in a continuous assay.

### BRIEF SUMMARY

In one aspect of the invention, there is a method of quantifying an HIV protease inhibitor in a biological sample, comprising combining HIV protease, a spectroscopic substrate for HIV protease, and a biological sample suspected of containing an HIV protease inhibitor to form an assay mixture; measuring a spectroscopic property of the assay mixture; and relating the spectroscopic property of the assay mixture to a concentration of the HIV protease inhibitor in the biological sample.

In another aspect of the invention, there is a method of monitoring a concentration of an HIV protease inhibitor in an organism, comprising administering a dose of an HIV protease inhibitor to an organism; obtaining a fluid sample from the organism; combining the sample with HIV protease and a spectroscopic substrate for HIV protease; measuring a spectroscopic property of the sample; and relating the spectroscopic property of the sample to the concentration of the HIV protease inhibitor in the organism.

In yet another aspect of the invention, there is a test kit, comprising a first container comprising HIV protease; a second container comprising a spectroscopic substrate for the HIV protease; and instructions to combine the HIV protease and the spectroscopic substrate with a biological sample, to measure a spectroscopic signal of the sample, and to relate the spectroscopic signal to a concentration of an HIV protease inhibitor in the sample.

### BRIEF DESCRIPTION OF THE DRAWINGS

Figure 1 is a graph of the decrease in absorbance as a function of time for the cleavage of a chromogenic substrate by HIV protease.
Figure 2 is a graph of the rate of substrate cleavage by HIV protease as a function of chromogenic substrate.
Figure 3 is a graph of the reduction in activity of HIV protease as a function of saquinavir concentration.
Figure 4 is a graph of the reduction in activity of HIV protease as a function of ritonavir concentration.

### DETAILED DESCRIPTION

The present invention relates to an enzymatic assay system for determining the concentration of HIV protease inhibitor compounds and/or anti-HIV protease antibodies in a sample. The system includes HIV protease (HIV-PR) and a spectroscopic substrate that provides a detectable spectroscopic signal when the substrate is cleaved by HIV-PR. When HIV-PR and the spectroscopic substrate are contacted with a sample containing an HIV protease inhibitor compound and/or an anti-HIV protease antibody, the spectroscopic signal changes, and this change can be correlated to the concentration of the HIV protease inhibitor compound and/or anti-HIV protease antibody in the sample.

The term HIV protease inhibitor, sometimes designated "HIV-PI" includes both HIV protease inhibitor compounds and anti-HIV protease antibodies. Examples of HIV protease inhibitor compounds include peptides, organic compounds, organometallic compounds, and metallic complexes which can reduce or eliminate the activity of HIV protease. Specific examples of HIV protease inhibitor compounds include the compounds which are currently approved by the FDA for treatment of AIDS patients, namely amprenavir, indinavir, lopinavir, nelfinavir, ritonavir, and saquinavir. An anti-HIV protease antibody is an antibody which has a specific binding affinity for the HIV protease enzyme to the exclusion of other substances. The term includes polyclonal antibodies, monoclonal antibodies and antibody fragments.

A peptide is any compound formed by the linkage of two or more amino acids by amide (peptide) bonds, usually a polymer of α-amino acids in which the α-amino group of each amino acid residue (except the NH₂-terminal) is linked to the α-carboxyl group of the next residue in a linear chain. The terms peptide, polypeptide and poly(amino acid) are used synonymously herein to refer to this class of compounds without restriction as to size. The largest members of this class are referred to as proteins.

The term "biological sample" means a bodily fluid obtained from a living organism. A biological sample is preferably an aqueous mixture, for example, urine, whole blood, plasma, serum, saliva, semen, stool, sputum, cerebral spinal fluid, tears, mucus or the like, but preferably is plasma or serum, and more preferably is human plasma or human serum.

Any sample that is suspected of containing an HIV-PI can be analyzed by the method of the present invention. The sample can be pretreated if desired and can be prepared in any convenient medium that does not interfere with the assay. An aqueous medium is preferred.

A spectroscopic substrate is a substance that provides a detectable spectroscopic signal when contacted with HIV-PR. The spectroscopic signal may be, for example, an increase or decrease in the absorbance of radiation at a particular wavelength. The spectroscopic signal may be, for example, an increase or decrease in the emission of fluorescent or phosphorescent radiation at a particular wavelength. In another example, the spectroscopic signal may be a shift in the wavelength at which a maximum in an absorbance or emission spectrum is observed. Other detectable spectroscopic signals may include measurements of optical density and luminescence.

Spectroscopic substrates may be, for example, polypeptide chains which are similar to natural substrates for HIV-PR. HIV-PR is believed to be responsible for cleavage of at least nine distinct sites within the *gag* and *gag-pol* polypeptides. The HIV-PR cleavage site of a given substrate is represented by the formula:

P6-P5-P4-P3-P2-P1*P1'-P2'-P3'-P4'-P5'

where amino acids are numbered P1, P2, etc. from the scissile bond toward the amino (N) terminus of the peptide and P1', P2', etc. from the scissile bond toward the carboxyl (C) terminus. The position of the scissile bond, which is broken during the cleavage of the substrate, is designated by the "*" symbol. It has been reported that a minimum of seven amino acid residues, spanning the P4-P3' sites, provide for effective interaction with an extended series of sub-sites on the surface of the enzyme.

Spectroscopic substrates preferably contain from 7 to 11 amino acid residues and further contain a scissile bond cleavable only by HIV-PR. The preferred substrate has a sequence based on the -Leu * Ala- cleavage site of the naturally occurring peptide H-His-Lys-Ala-Arg-Val-Leu*Ala-Glu-Ala-Met-Ser-NH2 (SEQ ID NO:1), which is one of the junctions of capsid protein (CA) and nucleocapsid protein (NC) in the HIV polypeptide.

A spectroscopic substrate may contain one or more moieties which provide the spectroscopic signal that changes when the substrate is cleaved by HIV-PR. The moiety within a spectroscopic substrate which provides the spectroscopic signal can be a variety of moieties. For example, a chromogenic group can be used to generate a change in the ultraviolet and/or visible (UV-visible) absorbance of the substrate, which can be detected by UV-visible spectroscopy techniques. In another example, one or more fluorescent groups within the substrate can generate a change in fluorescence behavior upon cleavage of the substrate by HIV-PR, and this change can then be detected by fluorescence spectroscopy techniques. In yet another example, an isotope labeled group containing deuterium, carbon-13, phosphorus-31 or another stable isotope, can be used to generate a change in the radiomagnetic properties of the substrate, which can be detected by nuclear magnetic resonance (NMR) techniques. In yet another example, a luminogenic group such as a dioxetane or a luciferin can be used to generate a change in the luminescence of the substrate, which can be detected by a luminometer.

Examples of chromogenic groups include the ρ-nitrophenyl group, which can be incorporated into a peptide chain by inclusion of a *p*-nitrophenylalanine (Nph) residue. Peptides having a Nph residue at P1 or P1' can be particularly useful spectroscopic substrates for HIV-PR. The hydrolysis of a spectroscopic substrate with Nph at the P1 or P1' position can result in a change in UV-visible absorbance at a particular wavelength, which is proportional to the rate of cleavage. Further modifications in the amino acid sequence of a substrate can lead to changes in sensitivity to HIV-PR, solubility and environmental stability. The pH dependence of the change in the spectroscopic properties can also be altered by sequence modifications, including placement of a Nph residue at the PI' position and replacement of a readily oxidized methionine (Met) residue by a less oxidizable norleucine (Nle) residue.

Spectroscopic substrates based on the peptide of SEQ ID NO:1 and containing a chromogenic group are described, for example, in Nashed, N.T. et al. *Biochem. Biophys. Res. Commun.* **16**3, 1079 (1989); in Richards, A.D. et al. *J*. *Biol. Chem.* **265**(14), 7733 (1990); and in Tomaszek, Jr., T.A. et al. *Biochem. Biophys. Res. Commun.* **168**, 274 (1990); all of which are incorporated herein by reference. The sequences of some preferred spectroscopic substrates containing chromogenic groups, referred to as chromogenic substrates, are as follows: Ac-Lys-Ala-Ser-Gln-Asn*Nph-Pro-Val-Val-NH2 (SEQ ID NO:2) H-His-Lys-Ala-Arg-Val-Leu*Nph-Glu-Ala-Nle-Ser-NH2 (SEQ ID NO:3) H-Lys-Ala-Arg-Val-Nle*Nph-Glu-Ala-Nle-NH2 (SEQ ID NO:4) H-Lys-Ala-Arg-Val-Tyr*Nph-Glu-Ala-Nle-NH2 (SEQ ID NO:5) H-Ala-Arg-Val-Nle*Nph-Glu-Ala-Nle-NH2 (SEQ ID NO:6) H-Arg-Val-Nle*Nph-Glu-Ala-Nle-NH2 (SEQ ID NO:7) Ac-Arg-Lys-Ile-Leu*Nph-Leu-Asp-Gly-NH2 (SEQ ID NO:8) H-Lys-Ala-Val-Leu*Nph-Glu-Ala-Met-NH2 (SEQ ID NO:9) H-Lys-Ala-Val-Leu*Nph-Glu-Ala-Nle-Gly-NH2 (SEQ ID NO:10) H-Lys-Ala-Val-Tyr*Nph-Glu-Ala-Nle-NH2 (SEQ ID NO:11) H-Lys-Ala-Val-Met*Nph-Glu-Ala-Nle-NH2 (SEQ ID NO:12) H-Lys-Ala-Val-Nle*Nph-Glu-Ala-Nle-NH2 (SEQ ID NO:13) H-Lys-Ala-Val-Nle*Nph-Glu-Ala-Nle-Gly-NH2 (SEQ ID NO:14) H-Lys-Ala-Val-Phe*Nph-Glu-Ala-Nle-Gly-NH2 (SEQ ID NO:15) H-Lys-Ala-Val-Ile*Nph-Glu-Ala-Nle-NH2 (SEQ ID NO:16) H-Lys-Ala-Val-Val*Nph-Glu-Ala-Nle-NH2 (SEQ ID NO:17) Ac-Thr-Ile-Nle*Nph-Gln-Arg-NH2 (SEQ ID NO:18). The cleavage of these substrates in solution can be monitored by measuring the absorbance of the solution at a wavelength of 300 nanometers (nm).

The HIV-PR substrates for fluorescent systems, referred to as fluorogenic substrates, may include a fluorescence donor on one side of the scissile bond and a fluorescence acceptor on the other side of the scissile bond. A fluorescence donor is a moiety which, when excited by irradiation at one wavelength, can emit radiation through fluorescence at a different wavelength. A fluorescence acceptor is a moiety which can absorb the energy from the donor to prevent the fluorescent emission. Thus, little or no net fluorescence is detected when the donor and acceptor are localized on the same substrate. Once the substrate is cleaved, the fluorescent behavior of each component can be detected by standard fluorescence spectroscopy techniques, including the detection of fluorescent emission from the fluorescence donor.

Examples of fluorescence donors include fluorescein; fluorescein derivatives such as fluorescein isothiocyanate and substituted fluoresceins; 2-aminobenzoate; rhodamine; tryptophan; and naphthalene derivatives, such as 5-(2'-aminoethylamino)naphthalene sulfonate. Examples of fluorescence acceptors include benzyl-azo derivative groups such as 4-(4'-dimethylaminobenzeneazo)benzoyl.

Spectroscopic substrates for HIV-PR containing fluorescent groups are described, for example, in Toth, M.V. et al. *Int. J. Pept. Res. Protein* **36**, 544 (1990); in Rich, D.H. et al. *J. Med. Chem.* **35**, 3803 (1992); in Geoghegan, K.F. et al. *FEBS Lett.* **262**, 119 (1990); and in Krafft, G.A. et al. *Methods in Enzymology* **241**(6), 70 (1994); all of which are incorporated herein by reference. For example, the substrate Ac-Thr-Ile-Nle*Nph-Gln-Arg-NH2 (SEQ ID NO:18), can be converted into a fluorogenic substrate by replacement of the acetyl (Ac) end group with a 2-aminobenzoic acid residue (Abz) to yield the fluorogenic substrate Abz-Thr-Ile-Nle*Nph-Gln-Arg-NH2 (SEQ ID NO:19). Once this substrate is cleaved, excitation of the cleavage products by irradiation at 337 nm can produce fluorescent emission with a maximum at 390-449 nm.

Other examples of fluorogenic substrates include X-Ser-Gln-Asn-Tyr-Pro-Ile-Val-Gln-Y (SEQ ID NO:20), where X is a 4-(4-dimethylaminophenylazo)benzoic acid residue and Y is a 5-[(2-aminoethyl)amino]naphthalene-1-suiphonic acid residue, and Z-Ser-Gln-Asn-Tyr-Pro-Ile-Val-Trp (SEQ ID NO:21), where Z is a 5-dimethylaminonaphthalene-1-sulfonyl residue. Excitation of the cleavage products of the substrate having the amino acid sequence of SEQ ID NO:20 by irradiation at 340 nm can produce fluorescent emission with a maximum at 490 nm. Excitation of the cleavage products of the substrate having the amino acid sequence of SEQ ID NO:21 by irradiation at 290 nm can produce fluorescent emission with a maximum at 360 nm. A polypeptide having the amino acid sequence of SEQ ID NO:5 has also been reported as a useful fluorogenic substrate, with excitation of the cleavage products by irradiation at 277 nm producing fluorescent emission with a maximum at 306 nm (PCT Patent Application publication no. WO 99/67417).

Preferably, the interactions of HIV-PR and the spectroscopic substrates described above follow Michaelis-Menten kinetics. Kinetic values such as the Michaelis constant (Kₘ), the rate constant (k_{cat}), and the maximal reaction rate (Vₘₐₓ) for these substrates can be determined by a spectrophotometer on standard automation analyzers, such as a ROCHE DIAGNOSTICS COBAS FARA (ROCHE DIAGNOSTICS, Indianapolis, IN). Preferably, the kinetic values are determined by mixing active HIV-PR at a concentration of 30-180 nanomolar (nM) (5-30 international units (IU)) with a spectroscopic substrate at concentrations from 0-400 micromolar (µM). The enzyme may be HIV-1 protease or HIV-2 protease. Preferably, the enzyme is HIV-1 protease. For example, for the chromogenic substrates described above, the rate of cleavage is preferably correlated to a decrease in absorbance at 300-310 nm over a pH range of 2-5 and a temperature range of 25-30°C. For fluorogenic substrates, the rate of cleavage is preferably correlated to a decrease in the fluorescence emission upon excitation by irradiation at the appropriate wavelength.

The concentration of a particular HIV-PI is inversely proportional to the inhibition of HIV-PR activity. Therefore by monitoring the interaction between HIV-PR and a spectroscopic substrate in a sample, the concentration of HIV-Pl in the sample can be determined. A standard HIV-PR inhibition test may be performed by a spectrophotometer on a standard automated analyzer. Preferably, active HIV-PR at a concentration of 60-120 nM is mixed with a spectroscopic substrate at a concentration of 100-200% of the Kₘ value of the substrate, and the HIV-PI of interest at concentrations from 0-1000 nM. The resulting reduction in the rate of cleavage of the spectroscopic substrate can be determined by measuring the change in a spectroscopic property. The results of rate reduction measurements for a range of concentrations can be used as a calibration for determining the concentration of the HIV-PI of interest in a sample containing an unknown amount of the inhibitor.

Calibration material means any standard or reference material containing a known amount of the analyte to be measured. The sample suspected of containing the analyte and the calibration material are assayed under similar conditions. Analyte concentration is then calculated by comparing the results obtained for the unknown specimen with results obtained for the standard.

Once a spectroscopic analyzer has been calibrated for one or more HIV-PI's of interest, a sample having an unknown concentration of one or more of the HIV-PI's can be analyzed and the resulting spectroscopic signal correlated with the concentration of the HIV-PI(s) in the sample. Examples of spectroscopic analyzers include UV-visible spectrophotometers, fluorometers, luminometers, and NMR instruments. Preferably, the measured concentration of HIV-PI(s) in the sample has a value between the lowest and highest concentrations used for the calibration.

The method of the present invention can be used to quantify HIV-PI's in biological samples. Biological samples include fluid samples from an organism, for example, urine, whole blood, plasma, serum, saliva, semen, stool, sputum, cerebral spinal fluid, tears, mucus or the like. Such fluids typically include a variety of components in addition to the water and the HIV-PI of interest, including various peptides, lipids, salts, and other compounds and/or antibodies. These extraneous components can interfere with the interaction between HIV-PR and either or both of the spectroscopic substrate and the HIV-PI. For example, the substrate and/or the HIV-PI may adsorb to or react with one or more of the extraneous components. The HIV-PR may also nonspecifically cleave other peptides in the fluid. It is surprising and unexpected that, given the possibility of interference in assays of body fluids, HIV-PI's can be quantified in biological samples by the enzymatic assay of the present invention.

The method of the present invention may thus be used to monitor the levels of HIV protease inhibitor compounds in patients to whom therapeutic doses have been administered. Evaluation of the levels of HIV protease inhibitor compounds and correlation to positive or negative symptoms can be used to modify the treatment of AIDS patients. The measurements of HIV protease inhibitor compounds may also be used to evaluate the effectiveness of such compounds or of combinations of such compounds. The method of the present invention may also be used to monitor the levels of anti-HIV protease antibodies in patients. Determinations of the presence of the antibodies can be helpful in diagnosing infection by HIV, and determinations of the levels of the antibodies can be helpful in evaluating a patient's response to the virus or to antiviral therapies.

The use of the method of the present invention may be facilitated by providing a test kit for measuring the concentration of HIV-PI's. For example, a test kit may contain, in packaged combination, active HIV-PR and a spectroscopic substrate for the HIV-PR. Preferably the HIV-PR enzyme and the spectroscopic substrate are packaged separately to ensure that the substrate concentration in the kit is not reduced due to cleavage by the enzyme. The ingredients of the kit may be in liquid or in lyophilized form. Preferably the ingredients are provided in amounts such that the ratio of the reagents provides for substantial optimization of the method and assay. The kit may also contain one or more calibrators comprising a known amount of an HIV-PI. The kit may also contain instructions for carrying out the method of the present invention. For example, the kit may contain instructions to combine the HIV-PR and the spectroscopic substrate with a biological sample and to measure the spectroscopic signal from the combined sample. The instructions may also provide the appropriate formulas to allow for convenient calculation of the concentration of HlV-Pl's in the sample.

The test kit may be optimized for particular purposes. For example, the amount of the HIV-PR and the type and amount of spectroscopic substrate may be varied to provide optimal sensitivity to anti-HIV protease antibodies for the diagnosis of HIV infection. In another example, the amount of the HIV-PR and the type and amount of spectroscopic substrate may be varied to provide optimal sensitivity to one particular HIV protease inhibitor compound. In yet another example, the amount of the HIV-PR and the type and amount of spectroscopic substrate may be varied to provide optimal sensitivity to a combination of HIV protease inhibitor compounds, such that the individual concentrations of the compounds can be measured.

### EXAMPLES

The following examples are provided by way of illustration and should not be seen as limiting the scope of the present invention.

The synthetic chromogenic substrate, H-His-Lys-Ala-Arg-Val-Leu*Nph-Glu-Ala-Nle-Ser-NH₂ (SEQ ID NO:3) and the enzyme HIV-1 protease (0.65 milligrams per milliliter (mg/mL), 16 International Units per milligram (lU/mg)) were purchased from BACHEM AMERICAS (King of Prussia, PA).

### Example 1 - Monitoring of Chromogenic Substrate Hydrolysis

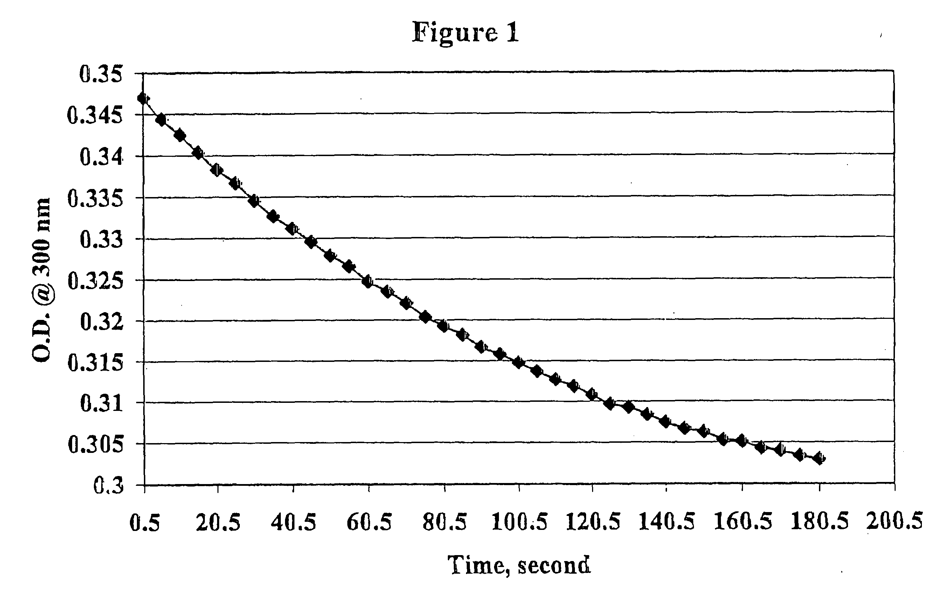

A stock solution containing 1 mg/mL of the chromogenic substrate in HIV PR assay buffer (50 millimolar (mM) sodium acetate, pH 4.9, 200 mM NaCI, 5 mM diothiothreitol (DTT), 10% (v/v) glycerol) was prepared by dissolving 5 mg chromogenic substrate in 5 mL of assay buffer. A substrate solution at a concentration of 101 micromolar (µM) was prepared in HIV-PR assay buffer from the substrate stock in a sample cup of Roche COBAS FARA. The stock mixture of HIV-1 PR as received was then diluted by 20-fold into this mixture. The hydrolysis of substrate by HIV-1 PR was monitored at 300 nm at 25°C on a ROCHE DIAGNOSTICS COBAS FARA instrument. The time course of hydrolysis is presented in Figure 1.

### Example 2 - Kinetic Parameter Determinations

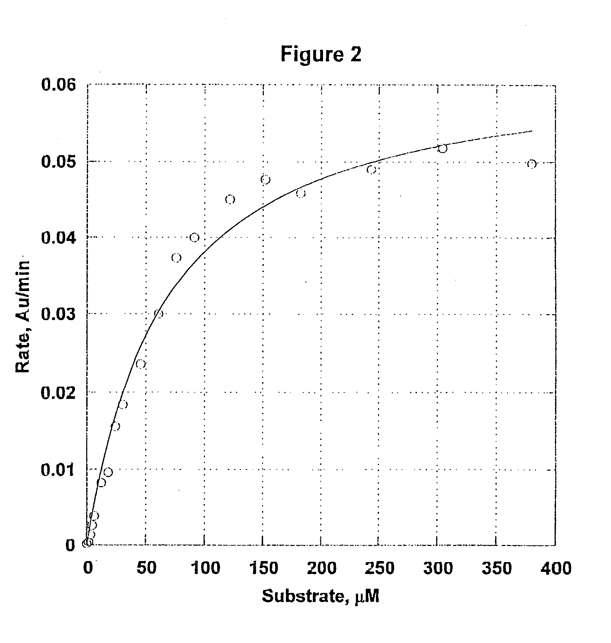

A stock solution containing the chromogenic substrate at a concentration of 3 mg/mL was prepared by dissolving 5 mg chromogenic substrate in 1.67 mL of the assay buffer as described in Example 1. A series of substrate solutions were prepared in the HIV-PR assay buffer over the concentration range of 0 - 2282 µM. The stock solution of HIV-1 PR was then diluted by 20-fold into HIV PR assay buffer. The hydrolysis of substrate by HIV-1 PR was monitored at 300 nm at 25°C on a ROCHE DIAGNOSTICS COBAS FARA instrument, as described in Example 1. The assay parameters on ROCHE COBAS FARA are shown in Table 1. A representative kinetic plot is presented in Figure 2. The substrate was hydrolyzed by HIV-1 PR with apparent Kₘ, Vₘₐₓ and k_{cat} values of 67.03 ± 7.55 µM, 156.81 micromoles per milligram minute (µmoles / mg·min) and 58.02 s⁻¹, respectively.

**Table 1.**

| Assay Parameters for ROCHE COBAS FARA Analysis | |
|---|---|
| Wavelength | 300 nm |
| Temperature | 25°C |
| Substrate Stock Solution | 20 microliters (µL) |
| Assay Buffer | 90 µL |
| HIV Protease I Stock Solution | 10 µL |
| Water Push | 0 |
| Reading | 0.5-180.5 sec |
| Reading Interval | 5 sec |
| Calculation | 0.5-30.5 sec |

### Example 3 - HIV-PR Inhibition Test With Saquinavir

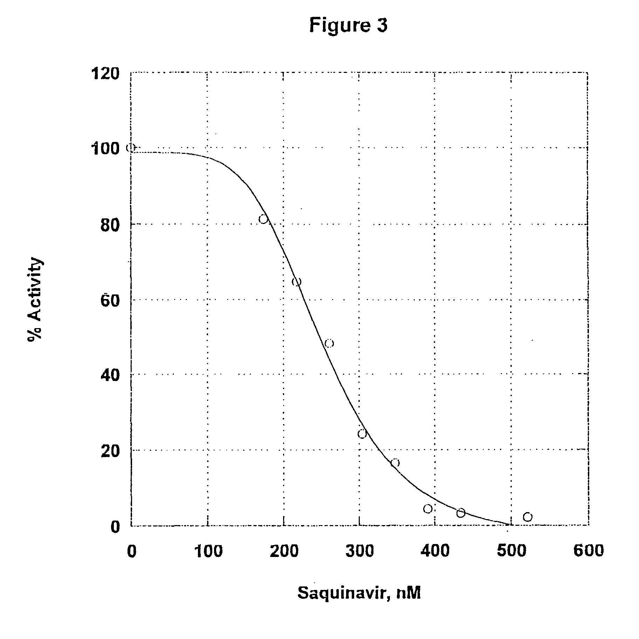

A stock solution of 1 mg/mL substrate was prepared as in Example 1 and then was added to HIV-PR assay buffer to yield a final substrate concentration of 67 µM. A stock solution of 1 mg/mL (13.04 µM) saquinavir was prepared by dissolving 1 mg saquinavir in 1 mL of methanol. The saquinavir stock was further diluted into a normal serum (ROCHE ZERO CALIBRATOR) to make a serum stock at concentration of 0.01 mg/mL. A series of saquinavir solutions were prepared from the serum stock in assay buffer over the concentration range of 0 - 522 nM. The stock solution of HIV-1 PR was diluted by 20-fold in the assay buffer. The hydrolysis of the chromogenic substrate by HIV-1 PR was monitored at 300 nm at 25°C on ROCHE COBAS FARA. The assay parameters on ROCHE COBAS FARA are shown in Table 1. A representative standard inhibition graph is presented in Figure 3.

### Example 4 - HIV-PR Inhibition Test With Ritonavir

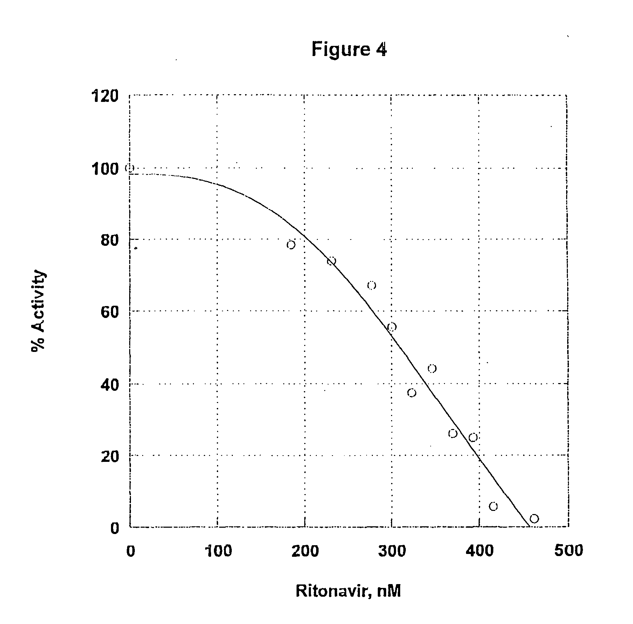

An inhibition test was performed as in Example 3, with the exception of using ritonavir instead of saquinavir. A stock solution of 1 mg/mL (13.87 µM) ritonavir was prepared by dissolving 1 mg ritonavir in 1 mL of dimethyl sulfoxide (DMSO). A series of ritonavir solutions were prepared from the serum stock in HIV-PR assay buffer over the concentration range of 0 - 462 nM. The assay parameters on ROCHE COBAS FARA are shown in Table 1. A representative standard inhibition graph is presented in Figure 4.

### Example 5 - Determination Of HIV-PI Levels In Control Subjects

A stock solution containing a synthetic chromogenic peptide substrate and HIV-1 protease are prepared as in Example 1. A stock solution containing protease inhibitors (indinavir sulfate, nelfinavir mesylate, ritonavir, and saqinavir) is prepared in methanol as described in Remmel et al. Different calibration solutions are prepared by dilution from the stock solution such that the final concentration ranges are 0.05-19.4 µg/ml for indinavir, 0.02- 8.6 µg/ml for nelfinavir, 0.05-20.0 µg/ml for ritonavir, and 0.02- 8.8 µg/ml for saquinavir (Remmel et al.). A quality control stock solution was prepared separately in methanol. Serum or plasma samples are obtained from the healthy human volunteers and are then supplemented with the HIV protease, a chromogenic substrate, and a portion of the stock solution of the HIV protease inhibitors. The concentration of the protease inhibitors is established by monitoring the hydrolysis of the chromogenic substrate by HIV protease in the serum or plasma samples by measuring the absorbance at 300nm at 25°C on a ROCHE COBAS FARA. Since the concentration of HIV protease inhibitors is inversely proportional to the decrease in the measured absorbance, the concentration of protease inhibitors in the serum or plasma is calculated.

### Example 6 - Determination Of HIV-PI Levels In AIDS Patients

A stock solution containing a synthetic chromogenic peptide substrate is prepared as in Example 1. Serum or plasma samples are obtained from AIDS patients 8 hours after treatment with an oral dose of protease inhibitors (Pl). The serum or plasma samples from AIDS patients are then supplemented with a chromogenic substrate and HIV-1 protease, and the concentration of HIV Pls is established by monitoring the hydrolysis of the chromogenic substrate by HIV protease in the serum or plasma samples by measuring the absorbance at 300nm at 25°C on a ROCHE COBAS FARA. Since the concentration of HIV protease inhibitors is inversely proportional to the decrease in the measured absorbance, the concentration of protease inhibitors in the serum or plasma is calculated. As a control, this assay is also performed without a chromogenic peptide substrate.

### Example 7 - Determination Of Anti-HIV Protease Antibody Levels In Biological Samples

A stock solution containing a synthetic chromogenic peptide substrate is prepared as in Example 1. Serum or plasma samples are obtained from AIDS patients within the first year from the AIDS diagnosis, and are then supplemented with a chromogenic substrate and HIV-1 protease. The concentration of the antibodies to HIV protease is measured by monitoring the hydrolysis of the chromogenic substrate by HIV protease in the serum or plasma samples by measuring the absorbance at 300nm at 25°C on a ROCHE COBAS FARA. Since the concentration of anti-HIV protease antibodies is inversely proportional to the decrease in the measured absorbance, the concentration of anti-HIV protease antibodies in the serum or plasma is calculated.

## Claims

1. A method of quantifying an HIV protease inhibitor in a biological sample, comprising:
combining HIV protease, a spectroscopic substrate for HIV protease, and a biological sample suspected of containing an HIV protease inhibitor to form an assay mixture;
measuring a spectroscopic property of the assay mixture; and
relating the spectroscopic property of the assay mixture to a concentration of the HIV protease inhibitor in the biological sample.

2. The method of claim 1, wherein the spectroscopic property comprises fluorescence emission.

3. The method of claim 1, wherein the spectroscopic property comprises UV-visible absorbance.

4. The method of claim 1, wherein the biological sample comprises human serum.

5. The method of claim 1, wherein the biological sample comprises human plasma.

6. The method of claim 1, wherein the HIV protease comprises HIV-1 protease.

7. The method of claim 1, wherein the HIV protease inhibitor comprises an HIV protease inhibitor compound.

8. The method of claim 7, wherein the inhibitor compound is selected from the group consisting of amprenavir, indinavir, lopinavir, nelfinavir, ritonavir, and saquinavir.

9. The method of claim 1, wherein the HIV protease inhibitor is an anti-HIV protease antibody.

10. The method of claim 2, wherein the spectroscopic substrate comprises a polypeptide having an amino acid sequence selected from the group consisting of SEQ ID NO:5, SEQ ID NO:19, SEQ ID NO:20 and SEQ ID NO:21.

11. The method of claim 3, wherein the spectroscopic substrate comprises a polypeptide having an amino acid sequence selected from the group consisting of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, and SEQ ID NO:18.

12. The method of claim 2, wherein the spectroscopic substrate comprises a polypeptide comprising a scissile bond between a first amino acid residue and a second amino acid residue;
wherein at least one of the first amino acid residue and the second amino acid residue is a *p*-nitrophenylalanine residue.

13. The method of claim 3, wherein the spectroscopic substrate comprises a polypeptide comprising a scissile bond between a first amino acid residue and a second amino acid residue;
wherein at least one of the first amino acid residue and the second amino acid residue is a *p*-nitrophenylalanine residue.

14. A method of monitoring a concentration of an HIV protease inhibitor in an organism, comprising:
administering a dose of an HIV protease inhibitor to an organism;
obtaining a fluid sample from the organism;
combining the sample with HIV protease and a spectroscopic substrate for HIV protease;
measuring a spectroscopic property of the sample; and
relating the spectroscopic property of the sample to the concentration of the HIV protease inhibitor in the organism.

15. The method of claim 14, wherein the HIV protease inhibitor comprises an HIV protease inhibitor compound selected from the group consisting of amprenavir, indinavir, lopinavir, nelfinavir, ritonavir, and saquinavir.

16. The method of claim 14, wherein the biological sample comprises human serum or human plasma.

17. The method of claim 14, wherein the HIV protease comprises HIV-1 protease.

18. The method of claim 14, wherein the spectroscopic property comprises UV-visible absorbance, and the spectroscopic substrate comprises a polypeptide having an amino acid sequence selected from the group consisting of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, and SEQ ID NO:18.

19. The method of claim 14, wherein the spectroscopic property comprises fluorescence emission, and the spectroscopic substrate comprises a polypeptide having an amino acid sequence selected from the group consisting of SEQ ID NO:5, SEQ ID NO:19, SEQ ID NO:20 and SEQ ID NO:21.

20. The method of claim 14, wherein the spectroscopic substrate comprises a polypeptide comprising a scissile bond between a first amino acid residue and a second amino acid residue;
wherein at least one of the first amino acid residue and the second amino acid residue is a ρ-nitrophenylalanine residue.

21. A test kit, comprising:
a first container comprising HIV protease;
a second container comprising a spectroscopic substrate for the HIV protease; and
instructions to combine the HIV protease and the spectroscopic substrate with a biological sample, to measure a spectroscopic signal of the sample, and to relate the spectroscopic signal to a concentration of an HIV protease inhibitor in the sample.

22. The test kit of claim 21, wherein the HIV protease comprises HIV-1 protease.

23. The test kit of claim 14, wherein the spectroscopic substrate comprises a polypeptide having an amino acid sequence selected from the group consisting of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20 and SEQ ID NO:21.


SEQUENCE LISTING

<110>  F. Hoffmann-LA Roche AG and Roche Diagnostics GmbH

<120>  Enzymatic Assay Of HIV Protease Inhibitors

<130>  21341/SZ

<140>  10/200,096
<141>  2002-07-18

<160>  21

<170>  PatentIn version 3.1

<210>  1
<211>  11
<212>  PRT
<213>  Unknown

<220>
<223>  chemically synthesized peptide

<400>  1

His Lys Ala Arg Val Leu Ala Glu Ala Met Ser
1               5                   10


<210>  2
<211>  8
<212>  PRT
<213>  Artificial Sequence

<220>
<223>  chemically synthesized peptide with a chromogenic group

<220>
<221>  MISC_FEATURE
<222>  (5)..(6)
<223>  *Nph


<400>  2

Lys Ala Ser Gln Asn Pro Val Val
1               5


<210>  3
<211>  10
<212>  PRT

<213>  Artificial Sequence

<220>
<223>  chemically synthesized peptide with a chromogenic group

<220>
<221>  MISC_FEATURE
<222>  (6)..(7)
<223>  *Nph


<220>
<221>  MOD_RES
<222>  (9)..(9)
<223>  Nle


<400>  3

His Lys Ala Arg Val Leu Glu Ala Leu Ser
1               5                   10


<210>  4
<211>  8
<212>  PRT
<213>  Artificial Sequence

<220>
<223>  chemically synthesized peptide with a chromogenic group

<220>
<221>  MISC_FEATURE
<222>  (5)..(6)
<223>  *Nph


<220>
<221>  MOD_RES
<222>  (5)..(5)
<223>  Nle


<220>
<221>  MOD_RES
<222>  (8)..(8)
<223>  Nle


<400>  4

Lys Ala Arg Val Leu Glu Ala Leu

1               5


<210>  5
<211>  8
<212>  PRT
<213>  Artificial Sequence

<220>
<223>  chemically synthesized peptide with a chromogenic group

<220>
<221>  MISC_FEATURE
<222>  (5)..(6)
<223>  *Nph


<220>
<221>  MOD_RES
<222>  (8)..(8)
<223>  Nle


<400>  5

Lys Ala Arg Val Tyr Glu Ala Leu
1               5


<210>  6
<211>  7
<212>  PRT
<213>  Artificial Sequence

<220>
<223>  chemically synthesized peptide with a chromogenic group

<220>
<221>  MOD_RES
<222>  (4)..(4)
<223>  Nle


<220>
<221>  MISC_FEATURE
<222>  (4)..(5)
<223>  *Nph


<220>
<221>  MOD_RES
<222>  (7)..(7)

<223>  Nle

<400>  6

Ala Arg Val Leu Glu Ala Leu
1               5

<210>  7
<211>  6
<212>  PRT
<213>  Artificial Sequence

<220>
<223>  chemically synthesized peptide with a chromogenic group

<220>
<221>  MOD_RES
<222>  (3)..(3)
<223>  Nle

<220>
<221>  MISC_FEATURE
<222>  (3)..(4)
<223>  *Nph

<220>
<221>  MOD_RES
<222>  (6)..(6)
<223>  Nle

<400>  7

Arg Val Leu Glu Ala Leu
1               5

<210>  8
<211>  7
<212>  PRT
<213>  Artificial Sequence

<220>
<223>  chemically synthesized peptide with a chromogenic group

<220>
<221>  MISC_FEATURE
<222>  (4)..(5)

<223>  *Nph

<400>  8

Arg Lys Ile Leu Leu Asp Gly
1               5

<210>  9
<211>  7
<212>  PRT
<213>  Artificial Sequence

<220>
<223>  chemically synthesized peptide with a chromogenic group

<220>
<221>  MISC_FEATURE
<222>  (4)..(5)
<223>  *Nph

<400>  9

Lys Ala Val Leu Glu Ala Met
1               5

<210>  10
<211>  8
<212>  PRT
<213>  Artificial Sequence

<220>
<223>  chemically synthesized peptide with a chromogenic group

<220>
<221>  MISC_FEATURE
<222>  (4)..(5)
<223>  *Nph

<220>
<221>  MOD_RES
<222>  (7)..(7)
<223>  Nle

<400>  10

Lys Ala Val Leu Glu Ala Leu Gly

1               5


<210>  11
<211>  7
<212>  PRT
<213>  Artificial Sequence

<220>
<223>  chemically synthesized peptide with a chromogenic group

<220>
<221>  MISC_FEATURE
<222>  (4)..(5)
<223>  *Nph


<220>
<221>  MOD_RES
<222>  (7)..(7)
<223>  Nle


<400>  11

Lys Ala Val Tyr Glu Ala Leu
1               5


<210>  12
<211>  7
<212>  PRT
<213>  Artificial Sequence

<220>
<223>  chemically synthesized peptide with a chromogenic group

<220>
<221>  MISC_FEATURE
<222>  (4)..(5)
<223>  *Nph


<220>
<221>  MOD_RES
<222>  (7)..(7)
<223>  Nle


<400>  12

Lys Ala Val Met Glu Ala Leu

1               5


<210>  13
<211>  7
<212>  PRT
<213>  Artificial Sequence

<220>
<223>  chemically synthesized peptide with a chromogenic group

<220>
<221>  MOD_RES
<222>  (4)..(4)
<223>  Nle


<220>
<221>  MISC_FEATURE
<222>  (4)..(5)
<223>  *Nph


<220>
<221>  MOD_RES
<222>  (7)..(7)
<223>  Nle


<400>  13

Lys Ala Val Leu Glu Ala Leu
1               5


<210>  14
<211>  8
<212>  PRT
<213>  Artificial Sequence

<220>
<223>  chemically synthesized peptide with a chromogenic group

<220>
<221>  MOD_RES
<222>  (4)..(4)
<223>  Nle


<220>
<221>  MISC_FEATURE
<222>  (4)..(5)

<223>  *Nph

<220>
<221>  MOD_RES
<222>  (7)..(7)
<223>  Nle

<400>  14

Lys Ala Val Leu Glu Ala Leu Gly
1               5

<210>  15
<211>  8
<212>  PRT
<213>  Artificial Sequence

<220>
<223>  chemically synthesized peptide with a chromogenic group

<220>
<221>  MISC_FEATURE
<222>  (4)..(5)
<223>  *Nph

<220>
<221>  MOD_RES
<222>  (7)..(7)
<223>  Nle

<400>  15

Lys Ala Val Phe Glu Ala Leu Gly
1               5

<210>  16
<211>  7
<212>  PRT
<213>  Artificial Sequence

<220>
<223>  chemically synthesized peptide with a chromogenic group

<220>
<221>  MISC_FEATURE
<222>  (4)..(5)

<223>  *Nph

<220>
<221>  MOD_RES
<222>  (7)..(7)
<223>  Nle

<400>  16

Lys Ala Val Ile Glu Ala Leu
1               5

<210>  17
<211>  7
<212>  PRT
<213>  Artificial Sequence

<220>
<223>  chemically synthesized peptide with a chromogenic group

<220>
<221>  MISC_FEATURE
<222>  (4)..(5)
<223>  *Nph

<220>
<221>  MOD_RES
<222>  (7)..(7)
<223>  Nle

<400>  17

Lys Ala Val Val Glu Ala Leu
1               5

<210>  18
<211>  5
<212>  PRT
<213>  Artificial Sequence

<220>
<223>  chemically synthesized peptide with a chromogenic group

<220>
<221>  MOD_RES
<222>  (3)..(3)

<223>  Nle

<220>
<221>  MISC_FEATURE
<222>  (3)..(4)
<223>  *Nph

<400>  18

Thr Ile Leu Gln Arg
1               5

<210>  19
<211>  5
<212>  PRT
<213>  Artificial Sequence

<220>
<223>  chemically synthesized peptide with a fluorescent group

<220>
<221>  MOD_RES
<222>  (3)..(3)
<223>  Nle

<220>
<221>  MISC_FEATURE
<222>  (0)..(1)
<223>  Abz=2-aminobenzoic acid

<220>
<221>  MISC_FEATURE
<222>  (3)..(4)
<223>  *Nph

<400>  19

Thr Ile Leu Gln Arg
1               5

<210>  20
<211>  8
<212>  PRT
<213>  Artificial Sequence

<220>
<223>  chemically synthesized peptide with a fluorescent group

<220>
<221>  MISC_FEATURE
<222>  (0)..(1)
<223>  X=4-(4-dimethylaminophenylazo)benzoic acid


<220>
<221>  MISC_FEATURE
<222>  (8)..()
<223>  Y=5-[(2-aminoethyl)amino]naphthalene-1-sulphonic acid


<400>  20

Ser Gln Asn Tyr Pro Ile Val Gln
1               5


<210>  21
<211>  8
<212>  PRT
<213>  Artificial Sequence

<220>
<223>  chemically synthesized peptide with a fluorescent group

<220>
<221>  MISC_FEATURE
<222>  (0)..(1)
<223>  Z=5-dimethylaminonaphthalene-1-sulfonyl


<400>  21

Ser Gln Asn Tyr Pro Ile Val Trp
1               5